(19) Europäisches Patentamt
European Patent Office
Office européen des brevets

(11) Veröffentlichungsnummer: **0 318 847 B1**

(12) **EUROPÄISCHE PATENTSCHRIFT**

(45) Veröffentlichungstag der Patentschrift: **16.09.92**

(51) Int. Cl.[5]: **C07D 213/85**

(21) Anmeldenummer: **88119637.2**

(22) Anmeldetag: **25.11.88**

# (54) Verfahren zur Herstellung von Cyano-Pyridinen.

(30) Priorität: **04.12.87 DE 3741159**

(43) Veröffentlichungstag der Anmeldung:
**07.06.89 Patentblatt 89/23**

(45) Bekanntmachung des Hinweises auf die Patenterteilung:
**16.09.92 Patentblatt 92/38**

(84) Benannte Vertragsstaaten:
**BE CH DE FR GB LI NL**

(56) Entgegenhaltungen:
**EP-A- 0 131 887**
**EP-A- 0 232 182**
**US-A- 4 220 783**

**CHEMICAL ABSTRACTS, Band 70, 1969, Seite 350, Zusammenfassung Nr. 68075d, Columbus, Ohio, US; H. ROZSA et al.: "Synthesis of 2-methylnicotinic acid"**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Goetz, Norbert, Dr.**
**Schoefferstrasse 25**
**W-6520 Worms 1(DE)**

EP 0 318 847 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Cyano-Pyridinen durch katalytische Umsetzung von Gemischen aus Acrolein und Alkanalen bzw. Ketonen mit Ammoniak in Gegenwart von Zeolith-Katalysatoren.

Es ist bekannt, daß bei der Umsetzung von Acrolein mit Ammoniak in der Gasphase in Gegenwart von Katalysatoren 3-Methylpyridin entsteht. Als Katalysatoren dienen insbesondere bei Temperaturen von 550 bis 1200°C mit Sauerstoff vorbehandelte Verbindungen aus den Elementen Al, F und O, die zusätzlich mindestens ein Element der zweiten, dritten oder vierten Gruppe des Periodensystems (DE-OS 2 151 417) oder mindestens zwei Elemente der zweiten, vierten, fünften oder sechsten Gruppe des Periodensystems (DE-OS 2 224 160) oder mindestens ein Element der zweiten Hauptgruppe des Periodensystems (DE-OS 2 239 801) enthalten. Es ist auch bekannt, bei Ausführung der Umsetzung im Wirbelbett das Acrolein getrennt vom Ammoniak in die Wirbelschicht einzuspeisen (DE-OS 2 449 340). Nachteilig ist bei diesen Verfahren, daß neben 3-Methylpyridin in erheblichem Umfang Pyridin entsteht und die Ausbeute an 3-Methylpyridin unter 30 % liegt.

Es ist außerdem bekannt, zur Herstellung von 3-Methylpyridin Gemische von Acrolein und Propionaldehyd mit Ammoniak umzusetzen. Als Katalysatoren werden Aluminiumoxid, Siliciumoxid oder Siliciumoxid in Mischung mit 5 bis 50 % Aluminiumoxid, gegebenenfalls mit Zusätzen von Oxiden weiterer Elemente verwendet (FR-PS 1 273 826). Bei diesem Verfahren beträgt die Ausbeute an 3-Methylpyridin 53 %.

In DE-PS 2 703 070 wird durch Einsatz von hochdispersen Aluminiumsilikaten mit einer BET-Oberfläche von 200-800 $m^2/g$ die Ausbeute an 3-Methylpyridin im Vergleich zu den obenbeschrieben Verfahren bis auf 61 % gesteigert.

Aus DE-PS 2 819 196 ist die Herstellung von aromatisch und heteroaromatisch substituierten Pyridinen bekannt. An Verbindungen aus den Elementen Al, F und O, die bei 550-1200°C vorbehandelt wurden und die zusätzlich ein Element der 2., 3. oder 4. Gruppe des Periodensystems enthalten, werden Ausbeuten bis 65 % Phenylpyridin erhalten.

Auch Lanthan dotierte Molekularsiebe (DE-PS 2 023 158) sind nur mäßig wirksame Katalysatoren und ergeben daher nur geringe Ausbeuten. Nachteilig ist hierbei auch, daß die Reaktion lediglich auf den Einsatz von Acrolein beschränkt ist, in Gegenwart von Sauerstoff ausgeführt und ein Gemisch 43 mol-% Pyridin und 22 mol-% 3-Methylpyridin erhalten wird.

In US-PS 4 220 783 wird ein Verfahren zur Herstellung von Pyridin und Picolin am Alumosilikatzeolith ZSM 5 aus $C_{2-4}$-Aldehyden oder $C_{3-5}$-Ketonen mit $NH_3$ beschrieben. Die Reaktion erfolgt in Gegenwart von Methanol oder Wasser. Im Beisein von Formaldehyd wird mehr Pyridin gebildet. Der Katalysator desaktiviert sehr schnell; nach 0,7 h werden 93 % Umsatz und nach 3 h nur noch 78 % Umsatz gefunden. Auch die Ausbeute mit 7,7 % Pyridin und 59,6 % Picolin läßt zu wünschen übrig. Mit den anderen genannten Katalysatoren werden noch schlechtere Ausbeuten erhalten; es treten mehr Kohlenwasserstoffe und hochsiedende Produkte auf.

Aus E-PS 131 887 ist bekannt, daß acide Alumosilikatzeolithe vom Pentasiltyp mit einem Constraint Index von 1 bis 12 beim Einsatz im Wirbelbett für die Herstellung von Alkylpyridinen bessere Ergebnisse liefern als im Festbett. Bei der Umsetzung von Acetaldehyd mit Formaldehyd beträgt die höchste Gesamtausbeute an der Summe der erhaltenen Pyridine 89,8 %, wobei das Pyridin/ß-Picolin-Verhältnis gleich 2 : 1 ist. Dieser hohe Wert wird allerdings nur zwischen der ersten und zweiten Stunde der Reaktion gefunden. Die Reaktionstemperatur soll höher als 450°C liegen und die Verweilzeit am Katalysator mehr als 2,5 sec. betragen. Nachteile dieses Verfahrens sind, daß der Katalysator bereits nach 4 Stunden regeneriert werden muß und das Produktspektrum nicht konstant gehalten werden kann und die Reaktion im technisch aufwendigen Wirbelbettverfahren ausgeführt werden muß, um hohe Ausbeuten zu erhalten.

Es bestand nunmehr die Aufgabe, mit einer Cyano-Gruppe subsitutuierte Pyridine aus leicht zugänglichen Ausgangsstoffen selektiv in Gegenwart von Katalysatoren zu synthetisieren, die sich durch hohe Aktivität und lange Standzeit auszeichnen.

Es wurde nun gefunden, daß Cyano-Pyridine der Formel (I)

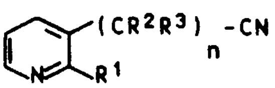

(I)

in guten Ausbeuten und bei langen Standzeiten der Katalysatoren aus leicht zugänglichen Ausgangsstoffen

durch Umsetzung von Acrolein und Alkanalen der Formel (II) bzw. deren Acetale und Ketonen der Formel (III)

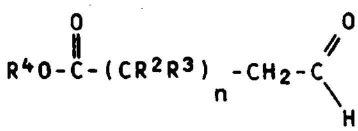 (II) 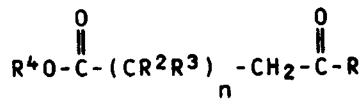 (III)

in der $R^1$, $R^2$ und $R^3$ untereinander gleich oder verschieden sein können und für Wasserstoff, Alkyl- mit 1-6 Kohlenstoffatomen oder Arylreste stehen und $R^4$ Alkylrest bedeutet und n = 0 - 11 ist, mit Ammoniak erhält, wenn man die Reaktion in Gegenwart von Zeolithen als Katalysatoren bei Temperaturen von 20-500 °C und Drücken von 0,1 bis 100 bar durchführt.

Die Reaktion wird vorzugsweise in der Gasphase bei einer Temperatur von 100 bis 500 °C ausgeführt. Geeignete Ausgangsverbindungen sind z.B. Acetessigsäuremethylester und Acetessigsäurepropylester.

Die Umsetzung läßt sich z.B. für den Fall von 2-Methyl-3-cyanopyridin durch folgende Formelgleichung wiedergeben:

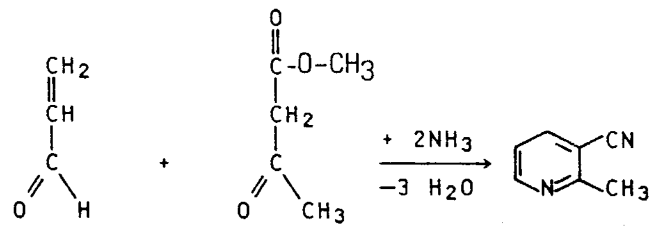


Als Katalysatoren für das erfindungsgemäße Verfahren werden acide zeolithische Katalysatoren eingesetzt. Zeolithe sind kristalline Aluminosilikate, die eine hochgeordnete Struktur mit einem starren dreidimensionalen Netzwerke von $SiO_4$- und $AlO_4$-Tetraedern besitzen, die durch gemeinsame Sauerstoffatome verbunden sind. Das Verhältnis der Si- und Al-Atome zu Sauerstoff beträgt 1 : 2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren verwendet man insbesondere Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabilen" Zeolithe des Faujasittyps, d.h. dealuminierte Zeolithe (US-PS 4 512 961).

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 200 ° C unter autogenem Druck hergestellt. Hierzu gehören auch die isotaktischen Zeolithe nach DE-OS 3 006 471 und EP 46 504. Die erhaltenen Aluminosilikatzeolithe enthalten je nach Wahl der Ausgangsstoffmengen ein $SiO_2/Al_2O_3$-Verhältnis von 10 bis 40 000. Diese Aluminosilikatzeolithe können auch in etherischem Medium, wie Diethylenglykoldimethylether, in alkoholischem Medium wie Methanol bzw. 1,4-Butandiol oder in Wasser synthetisiert werden.

Borosilikatzeolithe werden z.B. bei 90 bis 200 ° C unter autogenem Druck synthetisiert, indem man eine Borverbindung wie $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch die isotaktischen Zeolithe nach DE-OS 3 006 471 und EP 46 504 können verwendet werden. Solche Borosilikatzeolithe können auch hergestellt werden, indem man die Reaktion statt in wäßriger Aminlösung in etherischer Lösung, z.B. in Diethylenglykoldimethylether oder in alkoholischer Lösung, z.B. 1,6-Hexandiol durchführt.

Eisensilikatzeolithe erhält man aus einer Eisenverbindung, vorzugsweise $Fe_2(SO_4)_3$ und einer Siliciumverbindung, vorzugsweise hochdispersem Siliciumdioxid in wäßriger Aminlösung, insbesondere in 1,6-Hexandiamin, mit oder ohne Alkali- oder Erdalkalizusatz bei 100 bis 220 ° C unter autogenem Druck.

Zu den verwendbaren siliciumreichen Zeolithen ($SiO_2/Al_2O_3 \geqq 10$) gehören auch die sog. ZSM-Typen wie ZSM-5, ZSM-8, ZSM-12, ZSM-21, ZSM-22, ZSM-23, ZSM-25, ZSM-34, ZSM-35 und ZSM-48.

Auch Ferrierit als kristalliner Zeolith, und NU-1, sowie Silicalit®, ein Molekularsieb, ein sog. Silica Polymorph, sind geeignet.

Die so hergestellten Alumino-, Boro- und Eisensilikatzeolithe können nach ihrer Isolierung, Trocknung bei 100 bis 160 ° C, vorzugsweise 110 ° C und Calcinierung bei 450 bis 550 ° C, vorzugsweise 500 ° C, mit einem Bindemittel im Verhältnis 90 : 10 bis 40 : 60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 ° C/16 h getrocknet und bei 500 ° C/16 h calciniert.

Man erhält auch vorteilhafte Katalysatoren, wenn der isolierte Alumino- bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 ° C, insbesondere bei 500 ° C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4.-8. Nebengruppe wie Ti, Zr, V, Nb, Cr, Mo, W, Mn, Re, Fe, Ru, Os, Co, Rh, Ir, Ni, Pd, Pt, Übergangsmetalle der 1. und 2. Nebengruppe wie Cu, Ag, Zn, Seltene Erdmetalle wie La, Ce, Pr, Nd, Er, Yb und U eingesetzt.

Zweckmäßigerweise führt man die Dotierung so durch, daß man den verformten Zeolithen in einem Steigrohr vorlegt und bei 20 bis 100 ° C eine wäßrige oder ammoniakalische Lösung eines Halogenids oder eines Nitrats der obengenannten Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material mit einem Halogenid, einem Nitrat oder einem Oxid der Metalle in wäßriger, alkoholischer oder ammoniakalischer Lösung imprägniert. Sowohl an einen Ionenaustausch als auch an eine Imprägnierung schließt sich zumindest eine Trocknung,

wahlweise eine abermalige Calcinierung an.

Eine mögliche Ausführungsform besteht z.B. darin, daß man $Cu(NO_3)_2$ x 3 $H_2O$ oder $Ni(NO_3)_2$ x 6 $H_2O$ oder $Ce(NO_3)_3$ x 6 $H_2O$ oder $La(NO_3)_2$ x 6 $H_2O$ oder $Cs_2CO_3$ in Wasser löst. Mit dieser Lösung wird der verformte oder unverformte Zeolith eine gewisse Zeit, etwa 30 Minuten, getränkt. Die überstehende Lösung wird am Rotationsverdampfer von Wasser befreit. Danach wird der getränkte Zeolith bei etwa 150°C getrocknet und bei etwa 550°C calciniert. Dieser Tränkvorgang kann mehrmals hintereinander vorgenommen werden, um den gewünschten Metallgehalt einzustellen.

Es ist auch möglich, eine wäßrige $Ni(NO_3)_2$-Lösung oder ammoniakalische $Pd(NO_3)_2$-Lösung herzustellen und darin den reinen pulverförmigen Zeolithen bei 40 bis 100°C unter Rühren etwa 24 h aufzuschlämmen. Nach Abfiltrieren, Trocknen bei etwa 150°C und Calcinierung bei etwa 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber eine wäßrige $Ni(NO_3)_2$-Lösung oder ammoniakalische $Pd(NO_3)_2$-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert. Bei manchen metalldotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei kann man so vorgehen, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3 bis 25 Gew.%igen, insbesondere 12 bis 20 Gew.%igen wäßrigen Salzsäure. Anschließend wird der so behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Verformung bei erhöhter Temperatur mit 0,001 n bis 2 n, vorzugsweise 0,05 n bis 0,5 n Flußsäure, behandelt, beispielsweise durch Erhitzen unter Rückfluß über einen Zeitraum von 0,5 bis 5, vorzugsweise 1 bis 3 Stunden. Nach Isolierung, z.B. durch Abfiltrieren und Auswaschen, des zeolithischen Materials wird dieses zweckmäßig, bei Temperaturen von 100 bis 160°C, getrocknet und bei Temperaturen von 450°C bis 600°C calciniert. Gemäß einer weiteren bevorzugten Ausführungsform für die Säurebehandlung wird das zeolithische Material nach einer Verformung mit Bindemittel bei erhöhter Temperatur, zweckmäßig bei 50°C bis 90°C, insbesondere bei 60°C bis 80°C, über einen Zeitraum von 0,5 bis 5, vorzugsweise mit 12 bis 20 Gew.%iger Salzsäure, behandelt. Anschließend wird das zeolithische Material ausgewaschen und zweckmäßig bei Temperaturen von 100 bis 160°C, getrocknet und bei Temperaturen von 450 bis 600°C calciniert. Einer HF-Behandlung kann sich auch eine HCl-Behandlung anschließen.

Nach einer anderen Arbeitsweise kann man Zeolithe durch Aufbringung von Phosphorverbindungen, wie Trimethoxiphosphat, Trimethoxiphosphin, primärem, sekundärem oder tertiärem Natriumphosphat modifizieren. Als vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110°C getrocknet und bei 550°C calciniert.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Bei dem erfindungsgemäßen Verfahren wird zweckmäßig ein Molverhältnis von Acrolein : Alkanal bzw. Keton der Formel (II) bzw. (III) : $NH_3$ von 1 : 1 - 2 : 1 - 15 molar, bevorzugt 1 : 1 : 3 - 8 molar eingehalten. Die Umsetzung führt man vorteilhaft in der Gasphase bei Temperaturen von 100 bis 500°C durch. Vorteilhaft hält man eine Temperatur von 200 bis 450°C, insbesondere 250 bis 400°C ein. In der Regel führt man die Umsetzung bei einem Druck von 0,1 bis 100 bar, insbesondere 0,5 bis 10 bar durch.

Bei der Umsetzung von Acrolein und Alkanal der Formel (II) bzw. Keton der Formel (III) mit Ammoniak an den oben beschriebenen Katalysatoren in der Gasphase, hält man vorteilhaft eine Katalysatorbelastung von 0,1 bis 20, insbesondere von 1 bis 10 g Gemisch Acrolein und Alkanal/Keton der Formel (II), (III) je g Katalysator und Stunde ein (WHSV = g Einsatzgemisch/g Katalysator und Stunde).

Die Reaktionen in der Gasphase können in einem Festbett oder in einem Wirbelbett ausgeführt werden. Es ist auch möglich, die Reaktion in der Flüssigphase (Suspension-, Riesel- oder Sumpffahrweise) bei Temperaturen zwischen 20 und 200°C durchzuführen.

Die Durchführung der Reaktion kann diskontinuierlich, vorzugsweise kontinuierlich unter Normaldruck, Unterdruck oder Druck erfolgen.

Schwerflüchtige oder feste Ausgangsstoffe werden in gelöster Form, z.B. in THF-, Toluol- oder Petrolether-Lösung eingesetzt. Allgemein ist eine Verdünnung des Ausgangsstoffes mit derartigen Lösungsmitteln oder mit Inertgasen wie $N_2$, Ar, $H_2O$-Dampf möglich.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die erfindungsgemäße Umsetzung zurückgeführt.

Die nach dem erfindungsgemäßen Verfahren erhältlichen Cyano-Pyridine sind vielseitig verwendbare Zwischenprodukte.

Beispiele 1 bis 3

Die Reaktion in der Gasphase wird unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsprodukte erfolgt gaschromatographisch nach bekannter Methode.

Die für das erfindungsgemäße Verfahren eingesetzten Katalysatoren sind:

Katalysator A

Der Borosilikatzeolith des Pentasil-Typs wird in einer hydrothermalen Synthese aus 640 g hochdispersem $SiO_2$, 122 g $H_3BO_3$, 8 000 g einer wäßrigen 1,6-Hexandiamin-Lösung (Mischung 50 : 50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit Boehmit im Gewichtsverhältnis 60 : 40 2-mm-Stränge hergestellt, die bei 110 °C/16 h getrocknet und bei 500 °C/24 h calciniert werden.

Katalysator B

100 g des bei Katalysator A verwendeten Borosilikatzeolithen werden mit 280 ml einer 0,1 n HF bei 90 °C 2 h lang behandelt und nach Abfiltrieren bei 160 °C getrocknet. Dieses Produkt wird mit amorphem Aluminosilikat (25 Gew.% $Al_2O_3$ und 75 Gew.% $SiO_2$) im Gewichtsverhältnis 60 : 40 zu 2-mm-Strängen verformt, bei 110 °C/16 h getrocknet und bei 500 °C/16 h calciniert.

Katalysator C

Handelsüblicher NaY-Zeolith wird mit Boehmit im Gewichtsverhältnis 60 : 40 zu 2-mm-Strängen verformt, bei 110 °C/16 h getrocknet und bei 540 °C/24 h calciniert. Diese Stränge werden mit einer 20 %igen wäßrigen $La(NO_3)_2$-Lösung bei 80 °C/2 h ionenausgetauscht. Nach Trocknung bei 110 °C und Calcination bei 500 °C soll der La-Gehalt 7,1 Gew.% und der Na-Gehnalt 1,1 Gew.% betragen. Der Ionenaustausch kann nach Zwischencalcination wiederholt werden bis obige La- bzw. Na-Gehalte eingestellt sind.

Versuchsergebnisse und Reaktionsbedingungen sind in der folgenden Tabelle zusammengestellt. Nebenprodukte sind z.B. 2- und 3-Methylpyridin, 2-Methyl-nicotinsäuremethylester, 2-Methylnicotinsäureamid.

Tabelle

| Acrolein (I) + Acetessigsäuremethylester (II) + $NH_3$ → 2-Methyl-3-cyanopyridin (III) + $H_2O$ + $CH_3OH$ + $H_2$ | | | |
|---|---|---|---|
| Beispiel | 1 | 2 | 3 |
| Katalysator | A | B | C |
| Temperatur °C | 350 | 350 | 350 |
| WHSV $h^{-1}$ | 2 | 2 | 2 |
| morales Verhältnis | 1:1:6 | 1:1:6 | 1:1:6 |
| Umsatz % I + II | 100 | 100 | 100 |
| Selektivität % III | 56,7 | 61,5 | 48,9 |

## Patentansprüche

1. Verfahren zur Herstellung von Cyano-Pyridinen der Formel (I)

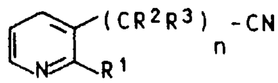

(I)

durch Umsetzung von Acrolein und Alkanalen der Formel (II) bzw. deren Acetale oder Ketonen der Formel (III)

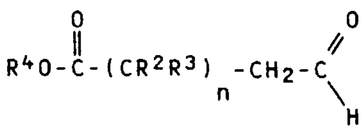

(II)

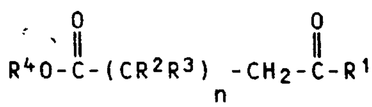

(III)

in der $R^1$, $R^2$ und $R^3$ gleich oder verschieden sein können und für Wasserstoff, Alkyl- mit 1 bis 6 Kohlenstoffatomen oder Arylreste stehen und $R^4$ Alkylrest bedeutet und n = 0 - 11 ist, mit Ammoniak, dadurch gekennzeichnet, daß man die Reaktion in Gegenwart von Zeolithen als Katalysatoren bei Temperaturen von 20 bis 500°C und Drücken von 0,1 bis 100 bar durchführt.

2. Verfahren nach Anspruch 1, dadurch gekennzeichnet, daß man Acetessigsäuremethylester einsetzt.

3. Verfahren nach den Ansprüchen 1 bis 2, dadurch gekennzeichnet, daß man als Katalysatoren Zeolithe des Pentasiltyps verwendet.

4. Verfahren nach den Ansprüchen 1 bis 3, dadurch gekennzeichnet, daß man als Katalysatoren Borsilikatzeolithe des Pentasiltyps verwendet.

5. Verfahren nach den Ansprüchen 1 bis 3, dadurch gekennzeichnet, daß man als Katalysatoren Eisensilikatzeolithe des Pentasiltyps verwendet.

6. Verfahren nach den Ansprüchen 1 bis 3, dadurch gekennzeichnet, daß man als Katalysatoren Aluminiumsilikatzeolithe des Pentasiltyps verwendet.

7. Verfahren nach den Ansprüchen 1 bis 2, dadurch gekennzeichnet, daß man als Katalysatoren Aluminiumsilikatzeolithe des Faujasittyps verwendet.

8. Verfahren nach den Ansprüchen 1 bis 7, dadurch gekennzeichnet, daß man als Katalysatoren Alkalimetallen oder mit Erdalkalimetallen oder mit Übergangsmetallen oder mit Seltenen Erden dotierte Zeolithe verwendet.

9. Verfahren nach den Ansprüchen 1 bis 8, dadurch gekennzeichnet, daß man die Umsetzung in der Gasphase durchführt.

## Claims

1. A process for the preparation of cyanopyridine of the formula (I)

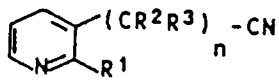

(I)

by reacting acrolein and an alkanal of the formula (II) or its acetal or ketone of the formula (III)

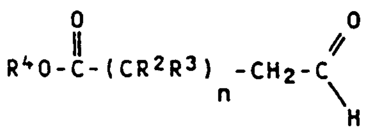

(II)

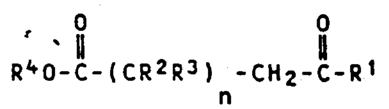

(III)

where $R^1$, $R^2$ and $R^3$ may be identical or different and are each hydrogen, alkyl or 1 to 6 carbon atoms or aryl and $R^4$ is alkyl and n is 0-11, with ammonia, wherein the reaction is carried out in the presence of a zeolite as a catalyst at from 20 to 500 ° C and under from 0.1 to 100 bar.

2. A process as claimed in claim 1, wherein methyl acetoacetate is used.

3. A process as claimed in claim 1 or 2, wherein the catalyst used is a zeolite of the pentasil type

4. A process as claimed in any of claims 1 to 3, wherein the catalyst used is a borosilicate zeolite of the pentasil type.

5. A process as claimed in any of claims 1 to 3, wherein the catalyst used is an iron zeolite of the pentasil type

6. A process as claimed in any of claims 1 to 3, wherein the catalyst used is an aluminosilicate zeolite of the pentosil type.

7. A process as claimed in claim 1 or 2, wherein the catalyst used is an aluminosilicate zeolite of the faujasite type

8. A process as claimed in any of claims 1 to 7, wherein the catalyst used is a zeolite which has been doped with alkali metals or with alkaline earth metals or with transition metals or with rare earth metals.

9. A process as claimed in any of claims 1 to 8, wherein the reaction is carried out in the gas phase.

## Revendications

1. Procédé de préparation de cyanopyridines de formule (I)

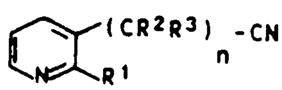

(I)

par réaction, avec de l'ammoniac, d'acroléine et d'alcanals de formule (II) ou de leurs acétals ou de cétones de formule (III)

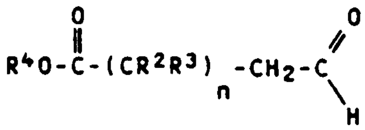 (II) 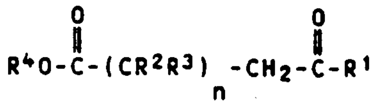 (III)

dans lesquelles $R^1$, $R^2$ et $R^3$ peuvent être identiques ou différents et sont mis pour des atomes d'hydrogène au pour des restes alkyle a 1-6 atomes de carbone ou aryle, $R^4$ représente un reste alkyle et n = 0-11, caractérisé en ce qu'on mène la réaction en présence de zéolithes servant de catalyseurs, à des températures de 20 a 500° C et sous des pressions de 0,1 à 100 bar.

**2.** Procédé selon la revendication 1, caractérisé en ce qu'on utilise de l'acétylacétate de méthyle.

**3.** Procédé selon la revendication 1 ou 2, caractérisé en ce qu'on utilise, comme catalyseurs, des zéolithes du type pentasil.

**4.** Procédé selon l'une quelconque des revendications 1 à 3, caractérisé en ce qu'on utilise, comme catalyseurs, des zéolithes de borosilicate du type pentasil.

**5.** Procédé selon l'une quelconque des revendications 1 à 3, caractérisé en ce qu'on utilise, comme catalyseurs, des zéolithes de ferrosilicate du type pentasil.

**6.** Procédé selon l'une quelconque des revendications 1 à 3, caractérisé en ce qu'on utilise, comme catalyseurs, des zéolithes d'aluminosilicate du type pentasil.

**7.** Procédé selon la revendication 1 ou 2, caractérisé en ce qu'on utilise, comme catalyseurs, des zéolithes d'aluminosilicate du type faujasite.

**8.** Procédé selon l'une quelconque des revendications 1 à 7, caractérisé en ce qu'on utilise, comme catalyseurs, des zéolithes dopées avec des métaux alcalins, avec des métaux alcalino-terreux, avec des métaux de transition ou avec des terres rares.

**9.** Procédé selon l'une quelconque des revendications 1 à 8, caractérisé en ce qu'on mène la réaction en phase gazeuse.